# EUROPEAN PATENT SPECIFICATION

(11) **EP 1 007 677 B1**
(45) Date of publication and mention of the grant of the patent: **24.11.2004**
(21) Application number: 98945221.4
(22) Date of filing: 12.08.1998
(51) Int. Cl.: C12N 15/12, C07K 14/47, C12N 1/21, C07K 16/18, G01N 33/68, A61K 38/17, A61K 48/00, A61K 39/395, A61K 31/70

(54) **PROTEASE-RELATED PROTEIN**
PROTEASE-ÄHNLICHES PROTEIN
PROTEINE APPARENTEE AUX PROTEASES

(30) Priority: 20.08.1997 DE 19736198
(43) Date of publication of application: 14.06.2000
(73) Proprietor: Deutsches Krebsforschungszentrum Stiftung des öffentlichen Rechts, 69120 Heidelberg (DE)
(72) Inventor: DEAR, Terence, N., D-69123 Heidelberg (DE); BOEHM, Thomas, D-69126 Heidelberg (DE)
(74) Representative: Schüssler, Andrea, Dr.
(86) International application number: PCT/EP1998/005110
(87) International publication number: WO 1999/009156

(56) References cited:
- KUROOKA ET AL: "Rescue of the hairless phenotype in nude mice by transgenic insertion of the wild-type Hfh11 genomic locus" INTERNATIONAL IMMUNOLOGY, vol. 8, no. 6, 1996, pages 961-966, XP002093572 cited in the application
- NOZAKI ET AL: "The complete sequence of the gene encoding mouse cytokeratin 15" GENE, vol. 138, 1994, pages 197-200, XP002093573 cited in the application
- WINTER ET AL: "Sequence and Expression of Murine Type I Hair Keratins mHa2 and mHa3" EXPERIMENTAL CELL RESEARCH, vol. 212, 1994, pages 190-200, XP002093574 cited in the application

## Description

The present invention relates to a protease-related protein, a DNA encoding the same and a process for the preparation thereof. In addition, this invention concerns the use of the DNA and the protein as well as antibodies directed against the protein.

A hair anomaly is frequently due to an impaired keratinization of hair. It is known from investigations made with naked mice that the gene product of a gene referred to as whn is important for the keratinization of hair. This gene product is a transcription factor. However, its target genes are not known. In so far, it is not possible to interfere with the keratinization of hair. However, this would be desirable, particularly if the keratinization of the hair is impaired.

Therefore, it is the object of the present invention to provide a product by which the keratinization of hair can be investigated and optionally be regulated.

According to the invention this is achieved by the subject matters defined in the claims.

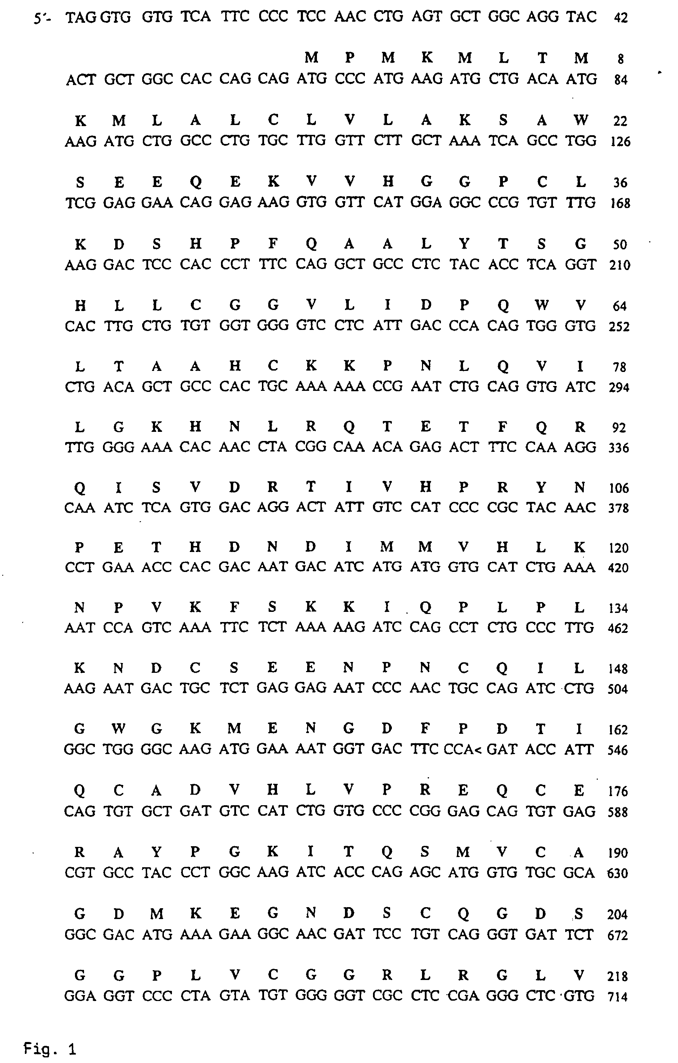

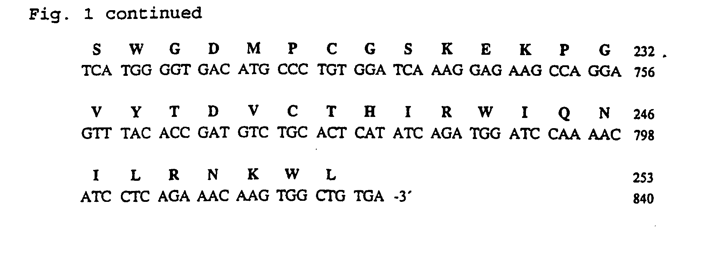

Thus, the subject matter of the present invention is represented by a protease-related protein, the protein comprising the amino acid sequence of fig. 1 or an amino acid sequence differing therefrom by one or more amino acids.

The present invention is based on the applicant's finding that the gene product of the whn gene is responsible for regulating the expression of at least three genes. Two of these genes code for the known keratins Ha3 (cf. Winter, H. et al., Exp. Cell Res. 212 (1994), 190-200) and CK15 (cf. Nozaki, M. et al., Gene 138 (1994), 197-200), respectively.

The third gene codes for a protein which has homologies with respect to a protease of the kallikrein family, optionally a protease activity, but differs from a known protease of the kallikrein family on the DNA level by hybridization under normal conditions. Such a protein has the amino acid sequence of fig. 1 or an amino acid sequence differing therefrom by one or more amino acids.

Kunvoka et al., Int. Immunol. 8(6) (1996), 961-966, Nozaki et al., Gene 138 (1994), 197-200, and Winter et al.) Exp. Cell. Res. 212 (1994), 190-200, are concerned with the whn gene and its effects, the Ck15 gene/protein and the Ha3 and Ha2 genes/proteins, respectively. However, these documents do not suggest, let alone disclose and characterize the specific protease-related protein of the present invention.

Furthermore, the applicant has found that when the gene product of the whn gene is lacking the genes of Ha3 and CK15 are underexpressed whereas the gene of the above protein is overexpressed.

The above protein is referred to as "protease-related protein" (PVP) in the present invention.

Another subject matter of the present invention relates to a nucleic acid coding for a (PVP). This may be an RNA or a DNA. The latter may be a genomic DNA or a cDNA, for example. Said DNA comprises the following:
(a) the DNA of fig. 1,
   or
(b) a DNA related to the DNA of (a) via the degenerated genetic code.

A section of the DNA of fig. 1 was deposited with the DSMZ (*Deutsche Sammlung von Mikroorganismen und Zellkulturen* [German-type collection of microorganisms and cell cultures]) as pRDA2-1a under DSM 11522 on April 23, 1997.

A DNA according to the invention is described below in the form of a cDNA. It stands as an example for every DNA falling under the present invention.

For the production of a cDNA according to the invention it is favorable to isolate mRNA from skin cells of whn(+/+) mice and nu/nu(whn(-/-)) mice, respectively, to transcribe the mRNA into cDNA and subject the cDNA to a "representational difference analysis" (RDA) method (cf. Hubank, M. and Schatz, D., Nucleic Acids Research 22 (1994), 5640-5648) so as to identify that cDNA which is underexpressed and overexpressed, respectively, in nu/nu mice as compared to whn(+/+) mice. In particular the latter cDNA represents a cDNA according to the invention.

A cDNA according to the invention may be present in a vector and expression vector, respectively. A person skilled in the art is familiar with examples thereof. In the case of an expression vector for E. coli, these are e.g. pGEMEX, pUC derivatives, pGEX-2T, pET3b and pQE-8. For the expression in yeast, pY100 and Ycpad1 have to be mentioned as examples, while for the expression in animal cells, e.g. pKCR, pEFBOS, cDM8 and pCEV4 have to be indicated. For the expression in insect cells, the baculo virus expression vector pAcSGHisNT-A is especially suitable.

The person skilled in the art is familiar with suitable cells to express a cDNA according to the invention, which is present in an expression vector. Examples of such cells comprise the E. coli strains HB101, DHl, x1776, JM101, JM 109, BL21 and SG 13009, the yeast strain *saccharomyces cerevisiae* and the animal cells Ltk, 3T3, FM3A, CHO, COS, Vero and HeLa as well as the insect cells sf9.

The person skilled in the art knows in which way a cDNA according to the invention has to be inserted in an expression vector. He is also familiar with the fact that this DNA can be inserted in connection with a DNA coding for another protein and peptide, respectively, so that the cDNA according to the invention can be expressed in the form of a fusion protein.

In addition, the person skilled in the art is familiar with conditions of cultivating transformed cells and transfected cells, respectively. He also knows processes serving for isolating and purifying the protein expressed by the cDNA according to the invention. Thus, such a protein which may also be a fusion protein represents a subject matter of the present invention as well.

Another subject matter of the present invention relates to an antibody directed against an above protein and fusion protein, respectively. Such an antibody may be prepared by conventional processes. It may be polyclonal and monoclonal, respectively. For its preparation it is favorable to immunize animals, particularly rabbits or chickens for a polyclonal antibody and mice for a monoclonal antibody, with an above (fusion) protein or fragments thereof. Further boosters of the animals can be effected with the same (fusion) protein or fragments thereof. The polyclonal antibody may then be obtained from the animals' serum and yolk, respectively. For the monoclonal antibody, spleen cells from the animals are fused with myeloma cells.

The present invention enables to investigate the keratinization of hair. (PVP) can be detected in cells, particularly skin cells, by means of an antibody according to the invention. A relation between (PVP) and the keratinization of hair can be established. Moreover, it is possible to detect by means of a (PVP) according to the invention an autoantibody directed against this protein. Both detections may be made by conventional processes, particularly a Western blot, an ELISA, an immunoprecipitation or by immunofluorescence. Furthermore, the expression of the gene coding for (PVP) can be detected by a nucleic acid according to the invention, particularly a DNA and primers derived therefrom. This detection can be carried out as usual, particularly in a Southern blot.

In addition, the present invention is suited to interfere in regulating fashion with the keratinization of hair. This regulation may be positive or negative. A positive regulation is understood to mean one by which an impaired keratinization of hair can be encountered. A negative regulation would exist if a normal or strong keratinization of hair was reduced.

For a positive regulation of the keratinization of hair it is an obvious thing to use (PVP) in the form of a substance inhibiting it. This substance may be an antibody according to the invention. Furthermore, it may be an anti-sense oligonucleotide which is suited for the expression inhibition of the gene coding for (PVP).

For a negative regulation of the keratinization of hair, it is an obvious thing to use (PVP) as such or in the form of a nucleic acid expressing it.

Thus, the present invention represents a major contribution to the understanding of the keratinization of hair and a possible regulating interference.

### Brief description of the drawing:

- Fig. 1: shows the base sequence of a cDNA according to the invention as well as the amino acid sequence, derived therefrom, of a (PVP) according to the invention.

The present invention is explained by the below examples.

### Example 1: Preparation of a cDNA according to the invention

A cDNA according to the invention was prepared according to the "representational difference analysis" (RDA) method. This method comprises the isolation of mRNA from skin cells of whn(+/+) mice and nu/nu mice, respectively, the transcription of the mRNA into cDNA, and the differentiation of the cDNA so as to identify that which is underexpressed and overexpressed, respectively, in nu/nu mice. In particular, the latter represents a cDNA according to the invention.

### A) Sequence of the oligonucleotide adaptors

The following oligonucleotide adaptor pairs were required for the RDA:

R-Bgl-12: 5'-GATCTGCGGTGA-3'

R-Bgl-24: 5'-AGCACTCTCCAGCCTCTCACCGCA-3'

R-Bgl-12: 5'-GATCTGTTCATG-3'

R-Bgl-24: 5'-ACCGACGTCGACTATCCATGAACA-3'

N-Bgl-12: 5'-GATCTTCCCTCG-3'

N-Bgl-24: 5'-AGGCAACTGTGCTATCCGAGGAA-3'

### B) Preparation of poly A-mRNA from the tissues to be compared with one another

RNA was initially obtained from the skin of whn(+/+) mice and nu/nu mice, respectively, according to the "single step RNA extraction" method (Chomczynski and Sacchi, 1987). Thereafter, the poly A-mRNA fractions from the two RNA populations were isolated by means of dynabeads oligo(dT) according to the corresponding protocol from the Dynal company.

### C) Synthesis of double-stranded cDNA

The "ribo clone cDNA synthesis kit" from the company of Promega was used for the synthesis of double-stranded whn(+/+) cDNA and nu/nu cDNA, respectively. 4 µg of poly A-mRNA were used each to obtain about 2 µg cDNA.

### D) Difference analysis

1. Restriction digest of the double-stranded cDNAs
a) About 2 µg of each cDNA were digested in a 100 µl reaction batch by the restriction endonuclease Dpnll at 37°C for 2 h.
b) The reaction solutions were then extracted twice with a phenol/chloroform mixture (1:1) and once with 100 % chloroform.
c) The DNA included in the aqueous phases of the two reaction batches was admixed with 2 µg glycogen, 50 µl 10 M ammonium acetate and 650 µl 100 % ethanol each and precipitated on ice for 20 min.
   After 14 minutes of centrifugation at 4°C and with 14000 rpm, the supernatant was discarded and the DNA pellet was washed with 70 % ethanol. After another centrifugation and removal of the alcoholic phase, the dried DNA was resuspended in 20 µl TE buffer.

2. Ligation of the cDNAs to the R-Bgl oligonucleotide adaptor pair
a) A reaction vessel was used to combine the following:
   20 µl cut cDNA (total reaction batch from item D)1c)
   8 µg R-Bgl-24
   4 µg R-Bgl-12
   6 µl 10 x ligase buffer
   x µl water
   57 µl final volume
b) The reaction batch was heated in a thermocycler (Peltier Thermocycler PTC-200, MJ Research) to 50°C, kept at this temperature for 1 min and then cooled again to 10°C in the course of one hour (ramp rate: 0.1°C/9 sec).
c) After adding 3 µl T4 DNA ligase (1 U/µl) , the mixture was incubated at 16°C overnight.

3. Synthesis of "representations" of the cDNA populations to be compared with one another
a) For generating what is called "representations" of the ligated cDNAs, the volume of the ligation batches from item 2c) was initially supplemented by the addition of 140 µl water each to give 200 µl.
Then, 30 reactions of 200 µl each were prepared from this dilute solution per cDNA population (whn(+/+) skin and nu/nu skin, respectively).
The following reactants were added successively to such a batch:
143 µl water
20 µl 10x PCR buffer
20 µl 2 mM dNTPs
10 µl 25 mM Mg chloride
2 µl R-Bgl-24 (1 µg/µl)
4 µl dilute ligation batch

b) PCR:
3 min: 72°C
addition of 1 µl Taq-DNA polymerase (5 U/µl)
20 x: 5 min: 95°C
   3 min: 72°C
finally: cooling to 4°C.

c) For preparing the reaction solutions, 4 reaction batches each were combined in a vessel.

| | |
|---|---|
| extraction | 2 x with 700 µl phenol/chloroform (1:1) each, 1 x with chloroform 100 %; |
| precipitation | addition of 75 µl 3 M Na-acetate solution (pH 5.3) and 800 µl 2-propanol to each reaction vessel, 20 min on ice. |
| centrifugation | 14 min, 14000 rpm, 4°C. |

Washing of the DNA pellet with ethanol 70 % and resuspension in such an amount of water that a concentration of 0.5 µg/µl resulted.
4. Restriction digest of the "representations"
a) For removing the R-Bgl oligonucleotide adaptors, 300 µg of each representation (whn(+/+) skin and nu/nu skin, respectively) were subjected to a restriction digest. Following the addition of the below reactants, incubation was carried out at 37°C for 4 h:
600 µl cDNA representation (0.5 µg/µl)
140 µl 10 x Dpnll buffer
100 µl Dpnll (10 U/µl)
560 µl water.

b) The restriction digest batch was distributed to 2 vessels prior to its preparation.

| | |
|---|---|
| Extraction | 2 x phenol/chloroform (1:1), 1 x chloroform 100 %; |
| precipitation | addition of 70 µl 3 M Na-acetate (pH 5.3), 700 µl 2-propanol to each vessel, 20 min on ice; |
| centrifugation | 14 min, 14000 rpm, 4°C. |

Washing of the DNA pellet with ethanol 70 % and resuspension in such an amount of water that a concentration of 0.5 µg/µl resulted.
The resulting Dpnll-digested whn(+/+) skin-cDNA representation represented the driver-DNA population to be used in the subtractive hybridization.
5. Synthesis of the tester DNA population
a) 20 µg of the nu/nu skin-cDNA representation digested with Dpnll (= tester DNA) were separated electrophoretically in a TAE gel:
   40 µl tester DNA (0.5 µg/µl)
   50 µl TE buffer
   10 µl 10 x loading buffer
   were applied to a 1.2 % agarose TAE gel. A voltage was applied to the gel until the bromophenol blue component of the loading buffer had migrated about 2 cm.
b) Thereafter, the bands containing the representation DNA were cut out of the gel and eluted by means of the "agarose gel DNA extraction kit" from the company of Boehringer Mannheim.
   The DNA extracts were purified, so that a total of 60 µl of solution was obtained. The concentration of this solution was evaluated by electrophoresis of 5 µl in a 1 % agarose gel.
c) Finally, the tester DNA was ligated with the J oligonucleotide pair:
   2 µg tester DNA eluate
   6 µl 10 x ligase buffer
   4 µl J-Bgl-24 (2 µg/µl)
   4 µl J-Bgl-12 (1 µg/µl)
   x µl water
   57 µl final volume
d) Transfer of the reaction batch into thermocycler:
   1 min: 50°C
   cooling to 10°C within 1 h (ramp rate: 0.1°C/9 sec).
e) After adding 3 µl T4 DNA ligase (1 U/µl), incubation at 16°C overnight.
f) Adjusting the concentration of the tester DNA to approximately 10 ng/µl by the addition of 120 µl water.

6. Subtractive hybridization
a) 80 µl of driver DNA (40 µg) from step 4. and 40 µl (0.4 µg) of dilute tester DNA from step 5., ligated with J oligonucleotides, were combined in a reaction vessel and extracted 2 x with phenol/chloroform (1:1) and once with chloroform 100 %.
b) Precipitation by addition of 30 µl of 10 M ammonium acetate, 380 µl of ethanol 100 %; -70°C for 10 min.

| | |
|---|---|
| Centrifugation | 14 min, 14000 rpm, 4°C. |
| Then | 2 x washing of the pellet with ethanol 70 %, short centrifugation after every wash step; drying of the DNA pellet. |

c) The DNA was resuspended in 4 µl EE x3 buffer (30 mM EPPS, pH 8.0, at 20°C (Sigma company); 3 mM EDTA) - accompanied by pipetting off and on for about 2 min, then heating to 37°C for 5 min, short "vortexing" and finally combining the solution again on the vessel bottom by centrifugation. The solution was eventually covered with a layer consisting of 35 µl of mineral oil.
d) Transfer of the reaction batch into thermocycler:
5 min: 98°C,
cooling to 67°C and immediate addition of 1 µl
5 M NaCl to the DNA,
20 h of incubation at 67°C.

7. Synthesis of the first difference product
a) After removing the mineral oil as completely as possible, the DNA was diluted step-wise:
   1. addition of 8 µl TE (+ 5 µg/µl yeast RNA)
   2. addition of 25 µl TE - thereafter thorough mixing
   3. addition of 362 µl TE - vortex.
b) 4 PCRs were prepared for every subtractive hybridization. Per reaction:
   127 µl water
   20 µl 10 x buffer
   20 µl 2 mM dNTPs
   5 µl 25 mM Mg chloride
   20 µl dilute hybridization solution (from step 7a))
c) PCR program:
   3 min: 72°C
   addition of 1 µl Taq DNA polymerase (5 U/µl)
   5 min: 72°C
   addition of 2 µl primer J-Bgl-24 (1 µg/µl)
   10 x: 1 min: 95°C
      3 min: 70°C
   finally: 10 min: 72°C, then cooling to room temperature
d) The 4 reaction batches were combined in a 1.5 ml vessel.
   extraction: 2 x phenol/chloroform (1:1), 1 x chloroform 100%.
   After the addition of 2 µg glycogen carrier:
      Precipitation with 75 µl 3 M Na acetate (pH 5.3),
      800 µl 2-propanol, 20 min on ice.
      Centrifugation: 14 min, 14000 rpm, 4°C.
      Washing of the DNA pellet with ethanol 70 %.
      After drying of the DNA, resuspension in 40 µl water.
e) 20 µl of the resuspended DNA from d) were subjected to a "mung bean nuclease digest" (MBN):
   20 µl DNA
   4 µl 10 x mung bean nuclease buffer (NEB company)
   14 µl water
   2 µl mung bean nuclease (10 U/µl; NEB company)
   35 min, 30°C.
   The reaction was discontinued by the addition of 160 µl 50 mM Tris-HCl (pH 8.9) and 5 minutes of incubation at 98°C. Thereafter, the vessel was placed on ice up to the next step.
f) During the MBN incubation, 4 further PCRs were prepared (on ice):
   127 µl water
   20 µl 2 mM dNTPs
   10 µl 25 mM Mg chloride
   2 µl J-Bgl-24 (1 µg/µl)
   20 µl MBN-digested DNA.
g) PCR program:
   1 min: 95°C
   allowing to cool to 80°C, addition of 1 µl Taq DNA polymerase (5 U/µl),
   18 x: 1 min: 95°C
      3 min: 70°C,
   finally: 10 min: 72°C; allowing to cool to 4°C.
h) The 4 PCR batches were combined in a vessel.
   Extraction: 2 x phenol/chloroform (1:1), 1 x chloroform 100 %.
   Precipitation: 75 µl 3 M Na-acetate (pH 5.3), 800 µl 2-propanol, 20 min on ice.
   Centrifugation: 14 min, 14000 rpm, 4°C.
   washing of the DNA pellet with ethanol 70 %. Resuspension of the DNA in 100 µl water (resulting concentration: 0.5 µg/µl); the solution obtained in this way represented the first difference product.

8. Exchange of the oligonucleotide adaptors of the difference product
a) Removal of the oligonucleotide adaptors by restriction digest with Dpnll:
   40 µl difference product 1 (0.5 µg/µl)
   30 µl 10 x Dpnll buffer
   15 µl Dpnll (10 U/µl)
   215 µl water
   37°C for 2 h.
b) Preparing the reaction batch:
   Extraction: 2 x phenol/chloroform (1:1), 1 x chloroform 100 %.
   Precipitation: 33 µl 3 M Na-acetate (pH 5.3), 800 µl ethanol 100 %, 20 min, -20°C.
   Centrifugation: 14 min, 14000 rpm, 4°C.
   Washing of the pellet in ethanol 70 % and resuspension in 40 µl water.
c) Ligation of the difference product to N-Bgl oligonucleotide adaptor pair
   1 µl of the prepared DNA solution from step b) was diluted with 9 µl water to give a concentration of 50 ng/µl; 4 µl of this solution were used in the following reaction:
   4 µl Dpnll-digested difference product 1 (200 ng)
   6 µl 10 x ligase buffer
   2.5 µl N-Bgl-24 (3.5 µg/µl)
   2 µl N-Bgl-12 (2 µg/µl)
   42.5 µl water.
d) After the transfer of the reaction batch into thermocycler:
   1 min: 50°C,
   allowing to cool to 10°C within one hour (ramp rate: 0.1°C/9 sec).
e) After adding 3 µl T4 DNA ligase (1 µg/µl), incubation at 16°C overnight.

9. Synthesis of the 2nd difference product
The ligation batch from step 8e) was diluted by adding 100 µl water to a concentration of 1.25 ng/µl. 40 µl of this dilution (50 ng) were mixed with 80 µl driver DNA (see item 4.) and treated again according to steps 6. to 8. When the oligonucleotide adaptors (step 8.) were changed, the J-Bgl oligonucleotides were ligated in this case to the newly formed difference product 2.
10. Synthesis of the 3rd difference product
The concentration of the difference product 2 ligated with the J-Bgl oligos was reduced to a concentration of 1 ng/µl. 10 µl of this solution were diluted again with 990 µl water (+ 30 µg yeast-RNA), so that the concentration was then 10 pg/µl. The subtractive hybridization was carried out with 100 pg (10 µl) J-ligated difference product 2 and 40 µg (80 µl) driver DNA from step 4. As for the rest, the procedure was carried out as in the case of the 1st and 2nd difference products according to steps 6. to 8. The PCR according to the MBN reaction (item 7.g) formed an exception - here only 18 cycles in place of 22 ones were carried out.
11. Cloning of the 3rd difference product
The 3rd difference product was initially subjected to a restriction digest with Dpnll so as to remove the oligonucleotide adaptors. The reaction product was then applied to a TAE gel and separated electrophoretically. The separated DNA bands were cut out of the gel, the DNA was eluted and cloned into a vector cut with BamHI (pBS Not).
12. Characterization of the difference products
In order to confirm that the cloned DNA fragments were not method artifacts but sequences which were actually included in the investigated DNA representations, Southern analyses were carried out in which the investigated cDNA representations were hybridized with the radioactively labeled cloning products.
Thereafter, those DNA fragments which proved to be "real" difference products in the Southern analysis, were investigated by means of Northern hybridizations: RNAs from the investigated tissues (whn(+/+) skin-cDNA and nu/nu skin-cDNA) were blotted and hybridized with the radioactively labeled cloning products. By this, the differential expression of these sequences was confirmed in the investigated tissues. An analysis of the sequences yielded the cDNA of fig. 1 according to the invention.

### Example 2: Preparation and purification of a (PVP) according to the invention

For the preparation of a (PVP) according to the invention, the vector pBSNot-PVP of Example 1 is cleaved by BamHI, the DNA coding for (PVP) is isolated and inserted in the expression vector pQE-8 (Quiagen company) cleaved by BamHI. The expression plasmid pQ/PVP is obtained. Such a plasmid codes for a fusion protein comprising 6 histidine residues (N terminus partner) and the (PVP) of fig. 1 according to the invention (C terminus partner). pQ/PVP is used for transforming E. coli SG 13009 (cf. Gottesman, S. et al., J. Bacteriol. 148, (1981), 265-273). The bacteria are cultivated in an LB broth with 100 µg/ml ampicillin and 25 µg/ml kanamycin and induced with 60 µM isopropyl-β-D-thiogalactopyranoside (IPTG) for 4 h. The addition of 6 M guanidine hydrochloride serves for achieving lysis of the bacteria, thereafter a chromatography (Ni-NTA resin) is carried out with the lysate in the presence of 8 M urea corresponding to the instructions of the manufacturer (Quiagen company) of the chromatography material. The bound fusion protein is eluted in a buffer having pH 3.5. After its neutralization, the fusion protein is subjected to an 18 % SDS-polyacrylamide gel electrophoresis and dyed with Coomassie blue (cf. Thomas, J.O. and Kornberg, R.D., J. Mol. Biol. 149 (1975), 709-733).

In this way, a (fusion) protein according to the invention can be prepared in highly pure form.

### Example 3: Preparation and detection of an antibody according to the invention

A fusion protein of Example 1 according to the invention is subjected to an 18 % SDS-polyacrylamide gel electrophoresis. After dyeing the gel using 4 M sodium acetate, an about 25 kD long band is cut out of the gel and incubated in phosphate-buffered common salt solution. Gel pieces are sedimented before the protein concentration of the supernatant is determined by SDS-polyacrylamide gel electrophoresis, which is followed by Coomassie blue dyeing. Animals are immunized with the gel-purified fusion protein as follows:

### Immunization protocol for polyclonal antibodies in rabbits

35 µg gel-purified fusion protein are used per immunization in 0.7 ml PBS and 0.7 ml complete Freund's adjuvant and incomplete Freund's adjuvant, respectively.

| | |
|---|---|
| day 0 | 1st immunization (complete Freund's adjuvant) |
| day 14 | 2nd immunization (incomplete Freund's adjuvant: icFA) |
| day 28 | 3rd immunization (icFA) |
| day 56 | 4th immunization (icFA) |
| day 80 | bleeding to death |

The serum of the rabbit is tested in an immunoblot. For this purpose, a fusion protein of Example 1 according to the invention is subjected to SDS-polyacrylamide gel electrophoresis and transferred to a nitrocellulose filter (cf. Khyse-Andersen, J., J. Biochem. Biophys. Meth. 10 (1984), 203-209) . The Western blot analysis is carried out as described in Bock, C.-T. et al., Virus Genes 8 (1994), 215-229). For this purpose, the nitrocellulose filter is incubated with a first antibody at 37°C for 1 h. This antibody is the serum of the rabbit (1:10000 in PBS). After several wash steps using PBS, the nitrocellulose filter is incubated with a second antibody. This antibody is a monoclonal goat anti-rabbit-IgG antibody coupled with alkaline phosphatase (Dianova company) (1:5000) in PBS. A 30-minute incubation at 37°C is followed by several wash steps using PBS and then by the alkaline phosphatase detection reaction using developer solution (36 µM 5'-bromo-4-chloro-3-indolylphosphate, 400 µM nitro blue tetrazolium, 100 mM Tris-HCl, pH 9.5, 100 mM NaCl, 5 mM MgCl₂) at room temperature, until bands are visible.

In this way, the polyclonal antibodies according to the invention can be prepared.

### Immunization protocol for polyclonal antibodies in chickens

40 µg gel-purified fusion protein are used per immunization in 0.8 ml PBS and 0.8 ml complete Freund's adjuvant and incomplete Freund's adjuvant, respectively.

| | |
|---|---|
| day 0 | 1st immunization (complete Freund's adjuvant) |
| day 28 | 2nd immunization (incomplete Freund's adjuvant; icFA) |
| day 50 | 3rd immunization (icFA) |

Antibodies are extracted from yolk and tested in a Western blot. Polyclonal antibodies according to the invention are detected in this way.

### Immunization protocol for monoclonal mouse antibodies

12 µg gel-purified fusion protein are used per immunization in 0.25 ml PBS and 0.25 ml complete Freund's adjuvant and incomplete Freund's adjuvant, respectively. The fusion protein was dissolved in 0.5 ml (without adjuvant) in the 4th immunization.

| | |
|---|---|
| day 0 | 1st immunization (complete Freund's adjuvant) |
| day 28 | 2nd immunization (incomplete Freund's adjuvant; icFA) |
| day 56 | 3rd immunization (icFA) |
| day 84 | 4th immunization (PBS) |
| day 87 | fusion |

Supernatants of hybridomas are tested in a Western blot. Monoclonal antibodies according to the invention are detected in this way.

## Claims

1. A protease-related protein, the protein comprising the amino acid sequence of Fig. 1.

2. A DNA coding for a protein according to claim 1, wherein the DNA comprises:
(a) the DNA of Fig. 1; or
(b) a DNA related to the DNA of (a) via the degenerated genetic code.

3. An expression plasmid comprising the DNA according to claim 2.

4. A transformant containing the expression plasmid according to claim 3.

5. A process for the preparation of the protein according to claim 1, comprising the cultivation of the transformant according to claim 4 under suitable conditions.

6. Antibodies directed against the protein according to claim 1.

7. Use of the protein according to claim 1, the DNA according to claim 2 or the antibody according to claim 6 for the preparation of a diagnostic composition for detecting the keratinization of hair.

8. Use of the protein according to claim 1 for the preparation of a pharmaceutical preparation for the negative regulation of the keratinization of hair.

9. Use according to claim 8, wherein the protein is present as such or in the form of a nucleic acid expressing it.

10. Use of a substance inhibiting the protein according to claim 1 for the preparation of a pharmaceutical composition for the positive regulation of the keratinization of hair, wherein said substance, is an antibody according to claim 6 and/or an anti-sense oligonucleotide which inhibits the expression of the nucleic acid encoding the protein.

## Patentansprüche

1. Protease-verwandtes Protein, wobei das Protein die Aminosäuresequenz der Fig. 1 umfasst.

2. DNA, die für ein Protein nach Anspruch 1 kodiert, wobei die DNA umfasst:
(a) die DNA der Fig. 1; oder
(b) eine DNA, die über den degenerierten genetischen Code mit der DNA von (a) verwandt ist.

3. Expressionsplasmid, das die DNA nach Anspruch 2 umfasst.

4. Transformante, die das Expressionsplasmid nach Anspruch 3 enthält.

5. Verfahren zur Herstellung des Proteins nach Anspruch 1, umfassend die Kultivierung der Transformante nach Anspruch 4 unter geeigneten Bedingungen.

6. Antikörper gerichtet gegen das Protein nach Anspruch 1.

7. Verwendung des Proteins nach Anspruch 1, der DNA nach Anspruch 2 oder des Antikörpers nach Anspruch 6 zur Herstellung einer diagnostischen Zusammensetzung zum Feststellen der Keratinisierung von Haar.

8. Verwendung des Proteins nach Anspruch 1 zur Herstellung eines pharmazeutischen Präparats für die negative Regulation der Keratinisierung von Haar.

9. Verwendung nach Anspruch 8, wobei das Protein als solches oder in der Form der Nukleinsäure, die es exprimiert, vorhanden ist.

10. Verwendung einer Substanz, die das Protein nach Anspruch 1 inhibiert, zur Herstellung einer pharmazeutischen Zusammensetzung für die positive Regulation der Keratinisierung von Haar, wobei die Substanz ein Antikörper nach Anspruch 6 und/oder eine anti-sense Oligunukleotid, das die Expression der das Protein kodierenden Nukleinsäure inhibiert, ist.

## Revendications

1. Protéine apparentée aux protéases, la protéine comprenant la séquence d'acides aminés de la figure 1.

2. ADN codant pour une protéine selon la revendication 1, dans lequel l'ADN comprend :
(a) l'ADN de la figure 1 ; ou
(b) un ADN apparenté à l'ADN selon (a) via le code génétique dégénéré.

3. Plasmide d'expression comprenant l'ADN selon la revendication 2.

4. Transformant contenant le plasmide d'expression selon la revendication 3.

5. Procédé pour la préparation de la protéine selon la revendication 1, comprenant la mise en culture du transformant selon la revendication 4 dans des conditions appropriées.

6. Anticorps dirigés contre la protéine selon la revendication 1.

7. Utilisation de la protéine selon la revendication 1, de l'ADN selon la revendication 2 ou de l'anticorps selon la revendication 6 pour la préparation d'une composition de diagnostic destinée à détecter la kératinisation des cheveux.

8. Utilisation de la protéine selon la revendication 1 pour la préparation d'une préparation pharmaceutique destinée à la régulation négative de la kératinisation des cheveux.

9. Utilisation selon la revendication 8, dans laquelle la protéine est présente en tant que telle ou sous la forme d'un acide nucléique l'exprimant.

10. Utilisation d'une substance inhibant la protéine selon la revendication 1 pour la préparation d'une composition pharmaceutique destinée à la régulation positive de la kératinisation des cheveux, dans laquelle ladite substance est un anticorps selon la revendication 6 et/ou un oligonucléotide anti-sens qui inhibe l'expression de l'acide nucléide codant la protéine.
